# FASCICULE DE BREVET EUROPEEN

(11) **EP 2 324 361 B1**
(45) Date de publication et mention de la délivrance du brevet: **22.02.2012**
(21) Numéro de dépôt: 09804581.8
(22) Date de dépôt: 06.08.2009
(51) Int. Cl.: G01N 33/68, G01N 33/86, A61B 5/02

(54) **NOUVEAU PROCÉDÉ DE PRONOSTIC ASSOCIÉ AUX CAS D'HÉMORRAGIES POST-PARTUM**
NEUES PROGNOSEVERFAHREN IN ZUSAMMENHANG MIT POSTPARTALER HÄMORRHAGIE
NOVEL METHOD OF PROGNOSIS ASSOCIATED WITH CASES OF POSTPARTUM HAEMORRHAGE

(30) Priorité: 06.08.2008 FR 0855454
(43) Date de publication de la demande: 25.05.2011
(73) Titulaire: Assistance Publique Hôpitaux De Paris, 75004 Paris 4 (FR)
(72) Inventeur: MEBAZAA, Alexandre, F-92120 Montrouge (FR); GAYAT, Etienne, F-75019 Paris (FR); RESCHE-RIGON, Matthieu, F-75019 Paris (FR); MOREL, Olivier, F-75014 Paris (FR); FARGEAUDOU, Yann, 75013 Paris (FR); ROSSIGNOL, Matthias, F-75019 Paris (FR); PAYEN, Didier, F-75018 Paris (FR)
(74) Mandataire: Warcoin, Jacques
(86) Numéro de dépôt international: PCT/EP2009/060240
(87) Numéro de publication internationale: WO 2010/015690

(56) Documents cités:
- ROMANO F ET AL: "THE POINT OF NO RETURN IN POST-PARTUM HAEMORRHAGE" ITALIAN JOURNAL OF GYNAECOLOGY & OBSTETRICS, C I C EDIZIONI INTERNAZIONALI S.R.L, ITALY, vol. 20, no. 2, 1 avril 2008 (2008-04-01), pages 112-118, XP002522446 ISSN: 1121-8339
- DUCARME ET AL: "Prise en charge chirurgicale des hémorragies de la délivrance : étude retrospective" GYNECOLOGIE OBSTETRIQUE & FERTILITE, EDITIONS SCIENTIFIQUES ET MEDICALES ELSEVIER, vol. 35, no. 12, 26 novembre 2007 (2007-11-26), pages 1209-1214, XP022385849 ISSN: 1297-9589
- CRANE JOAN M G ET AL: "Maternal complications with placenta previa" AMERICAN JOURNAL OF PERINATOLOGY, vol. 17, no. 2, 2000, pages 101-105, XP008104789 ISSN: 0735-1631
- BAKSHI S ET AL: "Indications for and outcomes of emergency peripartum hysterectomy. A five-year review." THE JOURNAL OF REPRODUCTIVE MEDICINE SEP 2000, vol. 45, no. 9, septembre 2000 (2000-09), pages 733-737, XP008104812 ISSN: 0024-7758

### SEQUENCE LISTING

<110> AP-HP (Assistance Publique des Hopitaux de Paris)
   MEBAZAA, Alexandre
   GAYAT, Etienne
   RESCHE-RIGON, Matthieu
   MOREL, Olivier
   FARGEAUDOU, Yann
   ROSSIGNOL, Matthias
   PAYEN, Didier
<120> NOUVEAU PROCEDE DE PRONOSTIC ASSOCIE AUX CAS D'HEMORRAGIES POST-PARTUM
<130> 354976 D26813
<160> 1
<170> PatentIn version 3.3
<210> 1
   <211> 210
   <212> PRT
   <213> Homo sapiens
<400> 1

## Description

La présente invention concerne un nouveau procédé de pronostic associé aux cas d'hémorragies post-partum.

L'hémorragie post-partum, qui est définie comme la perte de plus de 500 ml de sang après un accouchement, est encore observée lors de près de 18% des naissances. L'incidence annuelle des hémorragies post-partum est ainsi de 14 millions de cas dans le monde ; lesquelles sont responsables de 124 000 décès maternels En France, l'incidence annuelle de ces hémorragies est elle de 70 000 à 80 000 cas qui sont responsables de près de 250 décès. A ce titre, un rapport français explique que près de 75% de ces décès mettraient en cause un défaut de prise en charge au regard des recommandations d'usage intégrant notamment le délai de transfusion, la procédure hémostatique, etc.

Toutefois, les causes de ces hémorragies étant multiples, il est difficile à ce jour de définir un protocole de prise en charge permettant de limiter les décès liés au suite des hémorragies post-partum.

Il existe donc un besoin urgent de développer un test prédictif permettant aux praticiens de mieux gérer la prise en charge des patientes souffrant d'hémorragie post-partum.

Après une analyse exhaustive de différentes cohortes de parturientes ayant subi des hémorragies post-partum, les inventeurs ont mis en évidence l'existence de différents facteurs de risque à partir desquels ils ont développé un score permettant de prédire la nécessité d'effectuer une procédure invasive en vue de stopper le saignement.

En conséquence, un premier objet de l'invention consiste en un procédé *in vitro* de pronostic d'un arrêt du saignement chez une patiente atteinte d'hémorragie post-partum en l'absence de procédure invasive, comprenant les étapes suivantes :
a) déterminer, à partir d'un échantillon biologique de ladite patiente, la concentration plasmatique de fibrinogène, la concentration sanguine de troponine ainsi que le temps de prothrombine ;
b) étudier au moins deux marqueurs cliniques de ladite patiente choisis dans le groupe constitué de la fréquence cardiaque et de la présence d'anomalies de placentation ;
c) calculer un score Z selon la formule suivante : Z= a+ b + c + d + e,
   i) où a est associé à l'existence d'anomalies de placentation chez ladite patiente, avec a valant 0 lorsque aucune anomalie de placentation n'est observée chez ladite patiente et a valant 1 lorsqu'une ou plus anomalies de placentation sont observées chez ladite patiente ;
   ii) où b est associé à la fréquence cardiaque de ladite patiente avec b valant 0 lorsque la fréquence cardiaque de ladite patiente est inférieure ou égale à 115 bpm (battements par minute) et b valant 1 lorsque la fréquence cardiaque est supérieure à 115 bpm ;
   iii) où c est associé à la concentration plasmatique de fibrinogène de ladite patiente avec c valant 0 lorsque ladite concentration est supérieure ou égale à 2 g/L et c valant 1 lorsque ladite concentration est inférieure à 2g/L ;
   iv) où d est associé à la concentration sanguine en troponine de ladite patiente avec d valant 0 lorsque ladite concentration est inférieure au seuil de détection et d valant 1 lorsque ladite concentration est supérieure ou égale à ce même seuil de détection ;
   v) où e est associé au temps de prothrombine (TP) de ladite patiente avec e valant 0 lorsque ledit temps de prothrombine est supérieur ou égal à 50% et e valant 1 lorsque ledit temps de prothrombine est inférieur à 50% ;
   où un score Z supérieur ou égal à une valeur de 2 est indicatif de la nécessité d'effectuer une procédure invasive en vue de stopper le saignement chez ladite patiente souffrant d'une hémorragie post-partum.

En effet, les inventeurs ont mis en évidence qu'un score de 2 ou plus était associé à une probabilité de plus de 70 % d'effectuer une procédure invasive. Plus spécifiquement, les inventeurs ont mis en évidence qu'un score de 2 est associé à une probabilité de près de 50 % d'effectuer une procédure invasive, un score de 3 est associé à une probabilité de plus de 75% et enfin un score de 4 est associé à une probabilité de l'ordre de 90% d'effectuer une procédure invasive.

Finalement, le procédé développé par les inventeurs permet de définir simplement et avec une importante sensibilité, les patientes souffrant d'hémorragies post-partum nécessitant une procédure invasive en vue de stopper le saignement et, ainsi, d'envisager une prise en charge la plus adaptée dans les meilleurs délais.

De préférence, on entend par « échantillon biologique », un échantillon sanguin.

On entend par « fibrinogène » ou "facteur I" une glycoprotéine du plasma sanguin qui se transforme en fibrine sous l'action de la thrombine lors de la coagulation sanguine. Cette glycoprotéine est soluble et présente à une concentration normale comprise entre 1.8 et 4,0 g/L dans le plasma humain. La concentration plasmatique de fibrinogène peut être déterminée simplement par des méthodes de mesure bien connues de l'homme du métier. A titre de méthode de mesure de la concentration de fibrinogène, on peut citer la méthode ELISA telle qu'utilisée dans le kit STA FIBRINOGEN (DIAGNOSTICA STAGO).

On entend par « troponine », un complexe protéique agissant dans la sensibilisation des cellules musculaires au calcium.

Par « seuil de détection » et pour la troponine, on entend une concentration supérieure à la concentration détectable par la méthode utilisée. A titre d'exemple, on entend par seuil de détection généralement une valeur de 1 pg/L, de préférence de 10 pg/L et de manière particulièrement préférée de 0,02 ng/L.

Ainsi, d vaut 0 lorsque ladite concentration est inférieure à 1 pg/L, de préférence inférieure à 10 pg/L, et de manière particulièrement préférée inférieure à 0,02 ng/L.

De préférence, la troponine est la troponine I.

On entend par« Troponine 1 » ou « TnI » la sous-unité protéique responsable de l'inhibition de la liaison entre la myosine et l'actine (en masquant le site de l'actine qui sert à la liaison avec la myosine).

De préférence encore, ladite troponine 1 est la troponine 1 cardiaque (SEQ ID NO : :1), laquelle présente une fonction inhibitrice permettant d'amorcer la décontraction musculaire.

La troponine et, notamment, la troponine I, sont normalement absentes dans le sang et leur détection dans le sang est consécutive d'un stress et leur concentration sanguine normale doit être considérée comme nulle. La détection de troponine ou de troponine 1 peut être réalisée à l'aide de méthodes bien connues de l'homme du métier. A titre d'exemple, on peut citer notamment l'immunofluorescence avec notamment les kits STAT Troponin-1 (Architect i2000SR Abbotte), TROPONIN I ELISA kit (CALBIOTECH), ou CLEARVIEW TROPONIN 1 (INVERNESS MEDICAL INTERNATIONAL).

Par « temps de prothrombine » ou « TP », on parle également de « temps de Quick », on fait référence au temps de coagulation d'un plasma sanguin citraté en présence d'un extrait de tissu humain, animal ou synthétique appelé thromboplastine, cytozyme ou thrombokinase qui comprend un ensemble d'enzymes nécessaires à la coagulation du sang et permet de transformer la prothrombine en thrombine. Plus spécifiquement, le temps de Quick correspond à ce temps de coagulation mesuré et exprimé en secondes par rapport au temps obtenu pour un plasma témoin (en fait, la moyenne d'une cinquantaine de patients normaux) et le temps de prothrombine correspond lui à une valeur en pourcentage obtenu en reportant le temps de Quick obtenu pour le plasma à tester sur la droite de Thivolle (obtenue en testant des dilutions successives d'un plasma témoin normal). A titre d'exemple, le temps de prothrombine du plasma normal est par définition de 100%, celui du plasma normal dilué au demi de 50%, et ce temps de prothrombine est normalement compris entre 70 et 100 %.

Ce temps de prothrombine peut être mesuré simplement à l'aide de très nombreux kits ou appareillages disponibles sur le marché tels que les kits STA NEOPLASTINE (DIAGNOSTICA STAGO) ou IMMEDIA™ PT System (FARALLON MEDICAL).

Toutefois, ce taux de prothrombine a le principal défaut de varier suivant le réactif utilisé, c'est pourquoi on préfère parfois se baser sur l'INR (International Normalized Ratio ou rapport international normalisé) pour pouvoir comparer des mesures répétées chez un même patient. L'INR qui ne présente pas d'unité, prend lui en compte la sensibilité du réactif thromboplastine selon un indice déterminé internationalement. A titre d'exemple, un temps de prothrombine inférieur à 50% correspond à un INR supérieur à 1,6 (pour un TP mesuré par le kit STA NEOPLASTINE (DIAGNOSTICO STAGO).

Par « anomalies de placentation », on entend une anomalie de l'insertion placentaire. A titre d'exemple d'anomalies de placentation, on peut citer le placenta praevia, le placenta accreta, le placenta increta et le placenta percreta.

Le placenta praevia est caractérisé par une insertion basse du placenta (segment inférieur de l'utérus), qui obstrue donc partiellement ou complètement la voie d'expulsion naturelle, constituant un obstacle mécanique à l'accouchement par voie vaginale.

Pour le placenta accreta, c'est le placenta qui adhère de façon pathologique sur la paroi de la cavité utérine, dans cette anomalie, la décidua disparaît et les villosités trophoblastiques s'insèrent directement sur la couche musculaire de la paroi utérine (le myomètre). Selon le degré de la pénétration des villosités trophoblastiques placentaires dans le myomètre, on décrit trois niveaux de gravité :
■ le placenta accreta : la pénétration des villosités trophoblastiques est limitée à la couche superficielle du myomètre ;
■ le placenta increta : les villosités trophoblastiques pénètrent dans l'épaisseur du myomètre ;
■ le placenta percreta : les villosités trophoblastiques traversent la totalité de l'épaisseur du myomètre pour atteindre la séreuse et même les organes avoisinants (en particulier la vessie).

Par « procédure invasive », on entend une chirurgie d'hémostase ou une embolisation artérielle invasive.

A titre de chirurgie d'hémostase, on peut citer l'hystérectomie, la ligature vasculaire et/ou le packing intra-abdominal. De telles opérations de chirurgie d'hémostase sont notamment décrite dans l'article de SERGENT et al. (Ann. Chir., vol.131(4), p236-43, 2006).

Le procédé selon l'invention pourra, en parallèle du calcul du score, comprendre une étape de détermination d'autres paramètres biologiques, comme la concentration sanguine en hémoglobine, lesquels paramètres n'interviennent pas dans le calcul du score.

La présente invention sera maintenant décrite plus en détail à l'aide des exemples qui illustrent l'invention sans en limiter aucunement le cadre.

### EXEMPLES

### 1) Elaboration d'un score d'hémorragie post-partum pour prédire la performance d'une procédure invasive :

Une cohorte de 257 patientes de 31 ans d'âge moyen (28-35) souffrant d'hémorragies post-partum ont été sélectionnées, lesquelles ont été admises entre le 1^{er} janvier 2004 et le 31 décembre 2005 à l'hôpital Lariboisière (Paris, France). Parmi ces 257 patientes, 30 d'entre elles étaient déjà initialement à l'hôpital Lariboisière et les 227 autres ont été admises consécutivement à leur transfert depuis un autre centre hospitalier après que les différents traitements administrés pour réduire les saignements aient fait preuve de leur inefficacité. Le temps moyen pour le transfert était de 4,8 heures (3,4-7,2), transfert durant lequel la majorité des parturientes (87%) ont reçu des infusions continues de sulprostone. Plus spécifiquement, 12 avaient alors subi une hystérectomie et 11 une ligature vasculaire.

Dès leur entrée dans le service, les patientes se sont vues prises en charge avec un suivi et une correction des désordres hémodynamiques immédiats mettant en danger ces dernières. La persistance et l'intensité du saignement du vagin et/ou du péritoine ont été évalués par un examen physique simultanément avec une ultrasonographie.

A l'admission, les parturientes ont révélées des signes sévères avec des niveaux d'hémoglobine de 9,2 g/L et d'hématocrite de 27% malgré une transfusion de deux culots globulaires et d'un plasma frais congelé en moyenne avant admission. Les parturientes montraient également d'autres signes d'hémorragie incluant une tachycardie (fréquence cardiaque moyenne de 107 bpm), une coagulopathie (temps de prothrombine (TP) moyen de 67%) et thrombocytopénie (nombre moyen de plaquettes de 118 000 par mm³). Ainsi, le score de SAPS II (« Simplified Acute Physiology Score ») était de 18 pour les 257 patients.

Ces premières analyses ont montré que la cause principale d'hémorragie post-partum était une atonie utérine (69%), des lésions du tractus génital (22%) et enfin des anomalies de placentation (8%).

En fonction des résultats obtenus, deux options thérapeutiques étaient envisageables 1) une procédure invasive pour contrôler le saignement génital incluant i) une chirurgie pour une hémostase chirurgicale ou ii) une angiographie avec une embolisation utérine ; et 2) si aucune des deux n'était indiquée immédiatement, dans la mesure où le saignement aurait été stoppé ou serait minimal, les parturientes seraient maintenues en observation.

Après l'évaluation initiale, 110 parturientes ont subies des procédures invasives hémostatiques en raison de saignements persistants, après lesquelles les parturientes ont été maintenues en unité de soins intensifs ou intermédiaires.

Ces procédures ont inclus 85 embolisations artérielles seules (majoritairement des artères utérines), 14 chirurgies d'hémostase seules, et 11 embolisations et chirurgies combinées. Les chirurgies d'hémostase (n=25) incluaient des hystérectomies, des ligatures vasculaires et des pansements péritonéaux, effectués de façon combinée ou non. Plus spécifiquement, les embolisations artérielles ont été effectuées 1,9 heures (1,2-2,5) après admission et les procédures invasives ont-elles été effectuées 2,1 heures (1,4-4,6) après admission.

Pour la suite de l'analyse, la cohorte de 257 patientes s'est vue subdivisée en sous-groupes selon que la patiente ait subie ou non une procédure invasive, une procédure invasive (PI) étant définie comme étant une chirurgie d'hémostase (hystérectomie et/ou ligature vasculaire et/ou packing intra-abdominal) et/ou une embolisation artérielle utérine. Ainsi, les 257 patientes ont été subdivisées en un groupe « PI » incluant les patientes nécessitant une PI et un groupe « suivi médical » (SM) incluant des patientes dont aucune n'a subi une des procédures mentionnées précédemment.

Pour les 110 parturientes du groupe PI, deux décès ont été à déplorer et le temps moyen de séjour des autres parturientes de ce groupe a été de 3,2 jours (2,3-6,2), le saignement ayant été stoppé chez toutes les parturientes et aucune ré-hospitalisation n'ayant été observé.

Pour les 147 parturientes du groupe SM, le saignement a été considéré comme faible aussi bien durant l'admission que consécutivement. Aucune mort n'a été à déplorer dans ce groupe et le temps moyen d'hospitalisation de ces patientes a été de 0,9 jour (0,65-2,1) avec aucune ré-hospitalisation n'a été à déplorer.

Les caractéristiques précises de ces deux sous-groupes par rapport à la cohorte globale sont détaillées dans le tableau I qui suit.

**Tableau I**

| | | Cohorte (n=257) | Sous-groupe SM | Sous-groupe PI |
|---|---|---|---|---|
| Embolisation artérielle (n=96) | Une session unique | 96 (37%) | - | 96 (37%) |
| | Nécessité d'une seconde session | 7 (2,7%) | - | 7 (2,7%) |
| Chirurgie d'hémostase (n=25) | Hystérectomie | 7 (2,8%) | - | 7 (2,8%) |
| | Packing intra-abdominal | 17 (6,7%) | - | 17 (6,7%) |
| | Ligature vasculaire | 6 (2,4%) | - | 6 (2,4%) |
| Sévérité et pronostic | SAPS II | 18 (12-24) | 15 (12-21) | 22 (16,5-32) |
| | APACHE II | 10 (6-13) | 7 (6-11) | 12 (8-16) |
| | Pronostic réanimation | 37 (14,4%) | 6 (4%) | 31 (28%) |
| | Durée de séjour (jours) | 1,97 (0,8-4,0) | 0,94 (0,65-2,1) | 3,22 (2,3-6,17) |
| | Mortalité | 2 (0,8%) | 0 (0%) | 2 (1,8%) |

Plus spécifiquement, les différents paramètres collectés sur les patientes préalablement à l'admission et lors de l'admission sont présentés respectivement dans les tableaux II et III suivants.

**Tableau II**

| | **Total (n=257)** | **Groupe SM (n=147)** | **Groupe PI (n=110)** | **Valeur p** |
|---|---|---|---|---|
| Age | 31 [29-35] | 31 [27-35] | 32 [30-36] | 0.019 |
| **Paramètres obstétricaux** | | | | |
| Primiparité | 109 (46) | 49 | 41 | 0.23 |
| Primigestité | 85 (35) | 39 | 29 | 0.51 |
| Hémorragie antérieure | 8 (3) | 4 | 2 | 0.48 |
| post partum | | | | |
| Fibrome utérin | 12 (5) | 9 (6) | 3 (3) | 0.37 |
| Utérus cicatriciel | 30 (12) | 14(8) | 16 (16) | 0.07 |
| Toxémie | 30 (12) | 18 (12) | 12 (12) | 0.92 |
| Terme (semaines) | 39 [37-40] | 39 [38-40] | 39 [37-40] | 0.042 |
| Gémélité | 15 (6) | 7 (6) | 8 (7) | 1 |
| Accouchement dans un hôpital universitaire | 57 (22) | 25 (15) | 32 (32) | 0.0016 |
| **Détails de l'accouchement** | | | | |
| Déclenchement de l'accouchement | 49 (20) | 37 (25) | 12 (13) | 0.034 |
| Accouchement vaginal | 175 (70) | 110 (75) | 65 (65) | 0.21 |
| Durée du travail (heures) | 5 [4-8] | 6 [4-8] | 4 [2-6] | 0.016 |
| Manoeuvres instrumentales | 51 (21) | 33 (22) | 18 (19) | 0.63 |
| Délivrance artificielle | 124 (49) | 71 (48) | 53 (53) | 0.31 |
| Révision utérine | 174 (70) | 106 (72) | 68 (68) | 0.57 |
| **Anesthésie obstétricale** | | | | 0.35 |
| - Anesthésie générale | 27 (10) | 13 (9) | 14 (14) | |
| - Anesthésie épidurale | 162 (65) | 98 (66) | 64 (62) | |
| - Anesthésie spinale | 30 (12) | 18 (12) | 12 (13) | |
| - Pas d'anesthésie | 31 (13) | 20 (14) | 11 (11) | |
| **Causes d'hémorragie** | | | | 0.0034 |
| - Atonie utérine primaire | 178 (69) | 109 (74) | 69 (61) | |
| - Lésion génital | 56 (22) | 34 (23) | 22 (20) | |
| - Anormalité placentaire | 20 (8) | 4 (3) | 16 (14) | |
| - Rupture utérine | 3 (1) | 0 (0) | 3 (3) | |
| **Intervention chirurgicale avant transfert** | | | | |
| - Examen vaginal (écarteur) | 90 (36) | 61 (41) | 29 (30) | 0.11 |
| - Hystérectomie | 12 (5) | 4 (3) | 8 (8) | 0.079 |
| - packing intra-abdominal | 5 (2) | 0 (0) | 5 (5) | 0.012 |
| - Ligature vasculaire | 11 (4) | 7 (5) | 4 (4) | 1 |
| **Suivi médical avant admission** | | | | |
| Infusion de sulprostone | 217 (87) | 131 (89) | 86 (85) | 0.44 |
| Catécholamine | 5 (2) | 0 (0) | 5 (5) | 0.011 |
| Ventilation mécanique | 35 (14) | 13 (9) | 22 (20) | 0.028 |
| Culot Globulaire | 1.8 +/-3.2 | 1.2+/-2.0 | 2.8+/-4.1 | 0.00042 |
| Plasma Frais Congelé | 1.0+/-2.4 | 0.6+/-1.7 | 1.6+/-3.1 | 0.0026 |
| UCP | 0.2+/-1.3 | 0.1+/-0.4 | 0.4+/-1.9 | 0.11 |
| Temps entre l'accouchement et l'admission (heures) | 5 [3-7] | 5 [4-7] | 4 [3-7] | |
| Durée du transport (heures) | 0.5 [0.2-0.7] | 0.5 [0.2-0.7] | 0.5 [0.2-0.7] | 1 |

**Tableau III**

| | **Total (n=257)** | **Groupe SM (n=147)** | **Groupe PI (n=110)** | **Valeur p** |
|---|---|---|---|---|
| Statut hémodynamique | | | | |
| PAS (Pression artérielle systolique) (mmHg) | 110 [95-120] | 110 [100-120] | 100 [87-115] | 0.00069 |
| PAD (Pression artérielle diastolique) (mmHg) | 55 [50-60] | 60 [50-65] | 50 [45-60] | 0.00084 |
| FC (fréquence cardiaque) (bpm) | 105 [90-120] | 100 [90-115] | 115 [100-130] | 1.6^{e}-05 |

| Valeurs biologiques | | | | |
|---|---|---|---|---|
| pH | 7.42 [7.37-7.44] | 7.43 [7.4-7.45] | 7.39 [7.32-7.43] | 1.9^{e}-06 |
| PaCO₂ (mmHg) | 32 [29-36] | 32 [29-35] | 33 [29.25-38] | 0.1 |
| PaO2 (mmHg) | 161 [110-220.8] | 138 [104-187] | 195 [144-247] | 9.1^{e}-06 |
| Bicarbonate (mmol/l) | 22 [21-24] | 23 [22-24] | 22 [20-23] | 3.8^{e}-05 |
| Protéines (g/L) | 42 [37-49] | 45 [40-51] | 38 [32.5-43] | 1.7^{e}-10 |
| Créatinine (µmol/l) | 58 [50-69] | 57.5 [50.75-67.25] | 59 [48.5-70.5] | 0.66 |
| Lactate (mmol/l) | 2.13 [1.56-2.76] | 1.91 [1.39-2.5] | 2.5 [1.92-3.73] | 6.8^{e}-07 |
| AST (Ul/ml) | 22 [18-31] | 23 [19.25-30] | 21 [16-31] | 0.094 |
| ALT (UI/ml) | 13 [10-17] | 12 [10-16] | 13 [10-18] | 0.13 |
| Troponine I (ng/ml) | 0 [0-0.2] | 0 [0-0.03] | 0.06 [0-0.4] | 1.6^{e}-07 |
| Bilirubine (mmol/l) | 7 [4-12] | 7 [4-11] | 8 [5-15] | 0.17 |
| Hb (g/dL) | 9.2 [7.9-10.3] | 9.5 [8.2-10.6] | 8.7 [7.0-9.9] | 0.0013 |
| Hématocrite (%) | 27 [23-30] | 27 [24-31] | 25 [20-30] | 0.00064 |
| Plaquettes (/mm³) | 118000 [80250-154000] | 130500 [97750-161200] | 92500 [63750-133000] | 4.5^{e}-05 |
| TP (%) | 69 [55-79] | 73 [64-85] | 58.5 [38-73] | 8.7^{e}-09 |
| Fibrinogène (g/L) | 2.34 [1.55-3.12] | 2.65 [2.08-3.46] | 1.8 [1.09-2.52] | 2.9^{e}-10 |
| Facteur V (%) | 49 [31-61] | 52 [39-66] | 41 [23-56] | 0.00057 |

Les résultats font apparaître que, parmi les paramètres démographiques et obstétricaux collectés préalablement à l'admission des patientes, peu de différences ont pu être identifié entre les groupes PI et SM (voir tableau II). Parmi les paramètres significatifs identifiés, on peut ainsi citer un âge plus avancé, un terme écourté, une durée du travail plus courte, un usage plus fréquent du déclenchement du travail, une moindre atonie utérine primaire et plus d'anormalités de placentation (placenta praevia, placenta accreta et placenta percreta) parmi les patientes du groupe PI par rapport à celle du groupe SM. En revanche, des différences importantes ont pu être observé dans les mesures hémodynamiques et biologiques entre les groupes PI et SM lors de l'admission (voir tableaux II et III). Les patientes du groupe PI ont ainsi montré un statut hémodynamique instable avec une PAS et une PAD basse, une fréquence cardiaque élevée, un faible niveau d'hémoglobine, un niveau important de troponine I plasmatique et une acidose métabolique malgré une transfusion importante de globules rouges et une administration plus importante de catécholamine. La coagulopathie était également plus importante dans le groupe PI avec une faible quantité de plaquettes, un faible TP, un faible niveau de facteur V et de fibrinogène bien que plus de patientes du groupe PI aient été transfusées par rapport à celles du groupe SM.

En vue d'identifier les paramètres pertinents pour élaborer un modèle prédictif de la performance invasive, ces différents paramètres ont été analysés plus avant. Le but de l'étude étant de définir au moins la nécessité d'une embolisation, d'un packing intra-abdominal, d'une hystérectomie ou d'une ligature vasculaire, une association marginale entre des variables seules et le pronostic a été déterminé par un test de WILCOXON pour des variables quantitatives et par un test exact de FISCHER.

Pour se faire, une régression logistique multiple a été utilisée pour déterminer un ensemble de variables indépendamment associée à chaque pronostic. Les variables associées avec une intervention à un niveau de 0,15 et avec moins de 5% de données manquantes ont été considérées dans le modèle multiple. Pour la validité interne clinique, les covariables continues ont été catégorisées, notamment la pression artérielle systolique (PAS) < à 90 mmHg, la pression artérielle diastolique (PAD) < à 55 mmHg, une fréquence cardiaque (FC) > à 115 battements par minutes (bpm), le temps de prothrombine (TP) < à 50%, une concentration en fibrinogène < à 2g/L, et un niveau de troponine 1 détectable ou non.

Finalement, l'analyse multivariable a permis d'identifier 5 facteurs indépendants prédictifs du groupe PI, lesquels facteurs étaient identifiables dès l'admission des patientes. Ces facteurs étaient les suivants :
1) anormalités de placentation (OR : 7,05 [2,26-22,03], p=0,0007)
2) fréquence cardiaque (FC) > 115 bpm (OR : 2,18 [1,03-4,62], p=0,04)
3) Temps de prothrombine < 50% (OR : 3,55 [1,38-9,17], p=0,008)
4) Fibrinogène < 2 g/L (OR : 2,75 [1,51-4,95], p=0,005)
5) Niveau détectable de troponine I (OR : 2,73 [1,51-4,95], p=0,0009)

Au regard de ces facteurs, il a été possible de développer un score prédictif pour une patiente donné pour lequel score une valeur de 1 est attribuée pour chacun des précédents dès lors qu'il est observé chez cette patiente. Pour une patiente donnée et en fonction des facteurs identifiés chez celle-ci, un score pourra donc être déterminé, lequel score présentera une valeur comprise entre 0 et 5 selon que l'on observe ou non l'un ou plusieurs des facteurs précédemment décrits.

Au final, il est ressortit des résultats obtenus qu'une patiente présentant au moins un score de deux ou plus présentait un risque supérieur à 70% de nécessiter une procédure invasive pour le stopper le saignement consécutif à une hémorragie post-partum.

### 2) Validation du modèle prédictif

Pour se faire, le modèle prédictif a été testé sur une nouvelle cohorte de 150 patientes souffrant d'hémorragies post-partum admises durant l'année 2007. Les différents paramètres mesurés à l'admission sur cette nouvelle cohorte par rapport à ceux de la précédente cohorte sont décrit dans le tableau IV.

**Tableau IV**

| | | |
|---|---|---|
| | Cohorte 2004/2005 (n=257) | Cohorte 2007 |

| **DETAILS OBSTETRICAUX** | | |
|---|---|---|
| Age | 31 (29-35) | 32 (28-36) |
| Primiparité | 109 (46) | 67 (44) |
| Primigestité | 85 (35) | 56 (37) |
| Toxémie | 30 (12) | 15 (11) |
| Provenance d'un hopital universitaire | 57 (22) | 51 (34) |
| Gémélarité | 15 (6) | 8 (5) |
| Terme (semaines) | 39 (37-40) | 39 (38-40) |
| **Détails de l'accouchement** | | |
| Accouchement vaginal | 175 (70) | 83 (56) |
| Manoeuvre instrumentale | 51 (21) | 20 (14) |
| Révision utérine | 174 (70) | 83 (55) |
| Cause de l'hémorragie | | |
| Atonie utérine primaire | 178 (69) | 110 (73) |
| Lésion du tractus génitale | 56 (22) | 28 (19) |
| Anormalités de placentation | 20 (8) | 11 (7) |
| Rupture utérine | 3 (1) | 1 (1) |
| **PRISE EN CHARGE AVANT TRANSFERT** | | |
| Temps avant transfert | 5 (3-7) | 4,8 (3,4-7,2) |
| **Chirurgie avant transfert** | | |
| Examen avec rétracteur vaginal | 90 (36) | 75 (50= |
| Hystérectomie | 12 (5) | 4 (3) |
| Ligature vasculaire | 11 (4) | 8 (5) |
| **Prise en charge médicale** | | |
| Infusion de sulprostone | 217 (87) | 141 (94) |
| Ventilation mécanique | 35 (14) | 15 (10) |
| Culot Globulaire | 1,8 ± 3,2 | 2,1 ± 2,8 |
| Plasma Frais Congelé | 1,0 ± 2,4 | 1,1 ± 2,3 |

| **VALEURS A L'ADMISSION** | | |
|---|---|---|
| **Hémodynamique** | | |
| PAS (mmHg) | 110 (95-120) | 120 (110-132) |
| PAD (mmHg) | 55 (50-60) | 70 (60-78) |
| FC (bpm) | 105 (90-120) | 100 (80-110) |
| **Valeurs biologiques** | | |
| Troponine I (ng/ml) | 0 (0-0,2) | 0 (0-0,04) |
| Hb (g/dL) | 9,2 (7,9-10,3) | 9,0 (7,5-10,3) |
| Plaquettes (par mm³) | 118 000 (80 250-154 000) | 130 000 (92 000-160 000) |
| TP (%) | 69 (55-79) | 67 (58-76) |
| Fibrinogène (g/L) | 2,34 (1,55-3,12) | 2,7 (1,91-3,28) |

| **PRISE EN CHARGE DANS LE CENTRE** | | |
|---|---|---|
| SAPS-2 | 18 (12-24) | 13 (9,5-20) |
| Embolisation artérielle | 96 (37%) | 37 (25%) |
| Seconde embolisation artérielle | 7 (2,7%) | 2 (2%) |
| **Chirurgie d'hémostase** | | |
| Hystérectomie | 7 (2,8) | 2 (1) |
| Ligature Vasculaire | 6 (2,4) | 2 (1) |
| Séjour en réanimation | 37 (14,4%) | 10 (7%) |
| Décès | 2 (0,8%) | 0 (0%) |

Les résultats ont montré que les caractéristiques de la cohorte 2007 sont similaires à celles de la cohorte précédentes avec toutefois plus d'examen au rétracteur vaginal et moins de procédure invasive par rapport à la cohorte précédente.

Le score préalablement développé a été utilisé sur cette nouvelle cohorte.

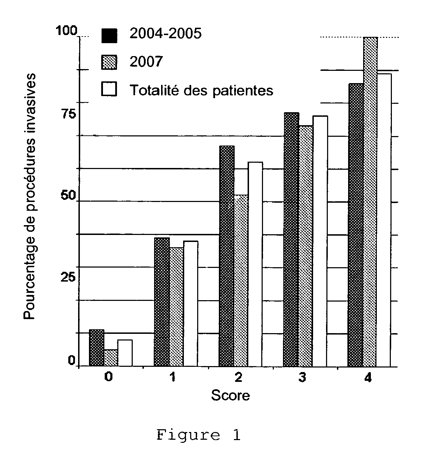

La figure 1 montre l'évolution du score tel que décrit précédemment au sein de la cohorte 2004/2005, la cohorte 2007 et de l'ensemble des patientes en fonction du risque de procédure invasive.

Finalement, les résultats obtenus ont permis de confirmer la spécificité importante du score tel que développé en tant qu'outil prédictif de la nécessité d'effectuer une procédure invasive pour stopper le saignement consécutif d'une hémorragie post-partum (voir figure 1).

## Revendications

1. Un procédé *in vitro* de pronostic d'un arrêt du saignement chez une patiente atteinte d'hémorragie post-partum en l'absence de procédure invasive, comprenant les étapes suivantes :
a) déterminer, à partir d'un échantillon biologique de ladite patiente, la concentration plasmatique de fibrinogène, la concentration sanguine de troponine ainsi que le temps de prothrombine ;
b) étudier au moins deux marqueurs cliniques de ladite patiente choisis dans le groupe constitué de la fréquence cardiaque et de la présence d'anomalies de placentation ;
c) calculer un score Z selon la formule suivante : Z= a+ b + c + d + e,
i) où a est associé à l'existence d'anomalies de placentation chez ladite patiente, avec a valant 0 lorsque aucune anomalie de placentation n'est observée chez ladite patiente et a valant 1 lorsqu'une ou plus anomalies de placentation sont observées chez ladite patiente ;
ii) où b est associé à la fréquence cardiaque de ladite patiente avec b valant 0 lorsque la fréquence cardiaque de ladite patiente est inférieure ou égale à 115 bpm (battements par minute) et b valant 1 lorsque la fréquence cardiaque est supérieure à 115 bpm ;
iii) où c est associé à la concentration plasmatique de fibrinogène de ladite patiente avec c valant 0 lorsque ladite concentration est supérieure ou égale à 2 g/L et c valant 1 lorsque ladite concentration est inférieure à 2g/L ;
iv) où d est associé à la concentration sanguine en troponine de ladite patiente avec d valant 0 lorsque ladite concentration est inférieure au seuil de détection et d valant 1 lorsque ladite concentration est supérieure ou égale au seuil de détection;
v) où e est associé au temps de prothrombine (TP) de ladite patiente avec e valant 0 lorsque ledit temps de prothrombine est supérieur ou égal à 50% et e valant 1 lorsque ledit temps de prothrombine est inférieur à 50% ;
où un score Z supérieur ou égal à une valeur de 2 est indicatif de la nécessité d'effectuer une procédure invasive en vue de stopper le saignement chez ladite patiente souffrant d'une hémorragie post-partum.

2. Le procédé selon la revendication 1, **caractérisé en ce que** ledit échantillon biologique est un échantillon sanguin.

3. Le procédé selon l'une quelconque des revendications 1 ou 2, **caractérisé en ce que** la troponine est la troponine I.

4. Le procédé selon la revendication 3, **caractérisé en ce que** la troponine I est la troponine I cardiaque.

5. Le procédé selon l'une quelconque des revendications 1 à 4, **caractérisé en ce que** d vaut 0 lorsque la concentration sanguine en troponine est inférieure à 1pg/L, de préférence inférieure à 10 pg/L.

6. Le procédé selon l'une quelconque des revendications 1 à 5, **caractérisé en ce que** les anomalies de placentation sont choisies dans le groupe comprenant ou consistant en le placenta praevia, le placenta accreta, le placenta increta et le placenta percreta.

7. Le procédé selon l'une quelconque des revendications 1 à 6, **caractérisé en ce que** ladite procédure invasive est une chirurgie d'hémostase ou une embolisation artérielle utérine.

8. Le procédé selon la revendication 7, **caractérisé en ce que** ladite procédure invasive est une chirurgie d'hémostase choisie dans le groupe comprenant une hystérectomie, une ligature vasculaire et un packing intra-abdominal.

9. Le procédé selon l'une quelconque des revendications 1 à 8, **caractérisé en ce qu'**il pourra inclure également la détermination d'autres paramètres biologiques.

## Claims

1. An *in vitro* prognosis method for halted bleeding in a patient suffering from postpartum haemorrhage without any invasive procedure, comprising the following steps :
a) determining, from a biological sample of said patient the plasmatic fibrinogen concentration, troponin blood concentration and prothrombin time ;
b) examining at least two clinical markers of said patient chosen from the group consisting of heart rate and the presence of placentation anomalies ;
c) calculating a Z score as per the following formula : Z = a + b + c + d + e,
i) where a is associated with the existence of placentation anomalies in said patient, with a having a value of 0 if no placental anomaly is observed in said patient, and a having a value of 1 if one or more placentation anomalies are observed in said patient;
ii) where b is associated with the heart rate of said patient, b having a value of 0 if the heart rate of said patient is less than or equal to 115 bpm (beats per minute) and b having a value of 1 if the heart rate is higher than 115 bpm;
iii) where c is associated with the plasmatic fibrinogen concentration of said patient, c having a value of 0 if said level is equal to or higher than 2 g/L and c having a value of 1 if said level is lower than 2 g/L;
iv) where d is associated with the blood troponin concentration of said patient, with d having a value of 0 when said level is lower than the detection threshold, and d having a value of 1 if said concentration is higher than or equal to the detection threshold;
v) where e is associated with the prothombin time (PT) of said patient, e having a value of 0 if said prothrombin time is greater than or equal to 50%, and e having a value of 1 if said prothrombin time is less than 50%;
wherein a Z score higher than or equal to a value of 2 is indicative of the need to carry out invasive procedure to stop bleeding in said patient suffering from postpartum haemorrhage.

2. The method according to claim 1, **characterized in that** said biological sample is a blood sample.

3. The method according to either of claims 1 or 2, **characterized in that** the troponin is troponin I.

4. The method according to claim 3, **characterized in that** troponin I is cardiac troponin I.

5. The method according to any of claims 1 to 4, **characterized in that** d has a value of 0 if the blood troponin concentration is lower than 1 pg/L, preferably lower than 10 pg/L.

6. The method according to any of claims 1 to 5, **characterized in that** the placental anomalies are chosen from the group comprising or consisting of *placenta praevia, placenta accreta, placenta increta* and *placenta percreta.*

7. The method according to any of claims 1 to 6, **characterized in that** said invasive procedure is haemostatic surgery or uterine arterial embolisation.

8. The method according to claim 7, **characterized in that** said invasive procedure is haemostatic surgery chosen from the group comprising hysterectomy, vessel ligature and intra-abdominal packing.

9. The method according to any of claims 1 to 8, **characterized in that** it may also include the determination of other biological parameters.

## Patentansprüche

1. *In vitro*-Verfahren für die Prognose über einen Blutungsstillstand bei einer Patientin mit einer postpartale Hämorrhagie ohne invasive Maßnahme, umfassend die folgenden Schritte:
a) Bestimmen der Fibrinogenplasmakonzentration, der Troponinkonzentration im Blut und der Prothrombinzeit anhand einer biologischen Probe der Patientin;
b) Untersuchen mindestens zweier klinischer Marker der Patientin ausgewählt aus der Gruppe bestehend aus der Herzfrequenz und dem Vorliegen von Anomalien der Plazentation;
c) Berechnen eines Wertes Z nach der folgenden Formel: Z = a + b + c + d + e,
i) wobei a für das Vorliegen von Anomalien der Plazentation bei der Patientin steht, und wobei a den Wert 0 hat, wenn bei der Patientin keine Plazentationsanomalie beobachtet wird, und a den Wert 1 hat, wenn eine oder mehrere Plazentationsanomalien bei der Patientin beobachtet werden;
ii) wobei b für die Herzfrequenz der Patientin steht, und wobei b den Wert 0 hat, wenn die Herzfrequenz der Patientin niedriger oder gleich 115 bpm (Herzschläge pro Minute) ist, und b den Wert 1 hat , wenn die Herzfrequenz höher als 115 bpm ist;
iii) wobei c für die Fibrinogenplasmakonzentration der Patientin steht, und wobei c den Wert 0 hat, wenn die Konzentration höher oder gleich 2 g/l ist, und c den Wert 1 hat, wenn die Konzentration niedriger als 2 g/l ist;
iv) wobei d für die Troponinkonzentration im Blut der Patientin steht, und wobei d den Wert 0 hat, wenn die Konzentration niedriger als der Nachweisgrenze ist, und d den Wert 1 hat, wenn die Konzentration höher oder gleich dem Nachweisgrenze ist;
v) wobei e für die Prothrombinzeit (PTZ) der Patientin steht, und wobei e den Wert 0 hat, wenn die Prothrombinzeit höher oder gleich 50% ist, und e den Wert 1 hat, wenn die Prothrombinzeit niedriger als 50% ist;
wobei ein Wert Z höher oder gleich einem Wert von 2 ein Indikator dafür ist, dass bei der Patientin mit postpartaler Hämorrhagie eine invasive Maßnahme zur Stillung der Blutung durchgeführt werden muss.

2. Verfahren nach Anspruch 1, **dadurch gekennzeichnet, dass** die biologische Probe eine Blutprobe ist.

3. Verfahren nach einem der Ansprüche 1 oder 2, **dadurch gekennzeichnet, dass** das Troponin Troponin I ist.

4. Verfahren nach Anspruch 3, **dadurch gekennzeichnet, dass** das Troponin I kardiales Troponin I ist.

5. Verfahren nach einem der Ansprüche 1 bis 4, **dadurch gekennzeichnet, dass** d den Wert 0 hat, wenn die Troponinkonzentration im Blut niedriger als 1 pg/l, bevorzugt niedriger als 10 pg/l ist.

6. Verfahren nach einem der Ansprüche 1 bis 5, **dadurch gekennzeichnet, dass** die Plazentationsanomalien ausgewählt sind aus der Gruppe umfassend oder bestehend aus Plazenta praevia, Plazenta accreta, Plazenta increta und Plazenta percreta.

7. Verfahren nach einem der Ansprüche 1 bis 6, **dadurch gekennzeichnet, dass** die invasive Maßnahme ein chirurgischer Eingriff der Hämostase oder eine Embolisation der Arteria uterina ist.

8. Verfahren nach Anspruch 7, **dadurch gekennzeichnet, dass** die invasive Maßnahme ein chirurgischer Eingriff der Hämostase ist, ausgewählt aus der Gruppe bestehend aus Hysterektomie, Gefäßligatur, intraabdominelles Packing.

9. Verfahren nach einem der Ansprüche 1 bis 8, **dadurch gekennzeichnet, dass** es ferner die Bestimmung weiterer biologischer Parameter einschließen kann.
